# EUROPEAN PATENT SPECIFICATION

(11) **EP 4 477 137 B1**
(45) Date of publication and mention of the grant of the patent: **29.07.2026**
(21) Application number: 23179803.4
(22) Date of filing: 16.06.2023
(51) Int. Cl.: A61B 5/00, A61B 5/318, A61B 5/349, G16H 50/50

(54) **ELECTROCARDIOGRAPHY BASED DETERMINATION OF A CARDIAC ACTIVATION AREA**
ELEKTROKARDIOGRAPHIEBASIERTE BESTIMMUNG EINES HERZAKTIVIERUNGSBEREICHS
DÉTERMINATION BASÉE SUR L'ÉLECTROCARDIOGRAPHIE D'UNE ZONE D'ACTIVATION CARDIAQUE

(43) Date of publication of application: 18.12.2024
(73) Proprietor: Varian Medical Systems Inc, Palo Alto CA 94304 (US)
(72) Inventor: PASSERINI, Tiziano, Plainsboro, 08536 (US); MEISTER, Felix, 91058 Erlangen (DE)
(74) Representative: Foster, Mark Charles

(56) References cited:
- WO-A1-2015/153832
- WO-A1-2019/145098
- KARLI GILLETTE ET AL: "MedalCare-XL: 16,900 healthy and pathological 12 lead ECGs obtained through electrophysiological simulations", ARXIV.ORG, CORNELL UNIVERSITY LIBRARY, 201 OLIN LIBRARY CORNELL UNIVERSITY ITHACA, NY 14853, 29 November 2022 (2022-11-29), XP091381478
- TRAYANOVA NATALIA A. ET AL: "How personalized heart modeling can help treatment of lethal arrhythmias: A focus on ventricular tachycardia ablation strategies in post-infarction patients", vol. 12, no. 3, 9 January 2020 (2020-01-09), pages e1477, XP055866209, Retrieved from the Internet <URL:http://dx.doi.org/10.1002/wsbm.l477> DOI: 10.1002/wsbm.l477

## Description

### FIELD OF THE INVENTION

The present invention relates generally to determining a cardiac activation area of a heart of a patient, and in particular to a method and a system for determining a cardiac activation area of a heart based on electrocardiography, e.g., for determining an area of ablation for ventricular tachycardia treatment.

### BACKGROUND OF THE INVENTION

Electrocardiography is a non-invasive medical means that may be used to represent and/or record the electrical activity of the heart of a patient. Electrocardiography is the process of producing an electrocardiogram, a recording of the heart's electrical activity through repeated cardiac cycles. An ECG apparatus measures electrical impulses generated by the heart and produces a visual representation of the heart's activity, called an electrocardiogram (ECG). The electrical impulses are detected by electrodes applied to the body of the patient. The electrodes detect electrical potentials at the corresponding positions where the electrodes are applied. The ECG includes several graphs that represent the detected electrical potentials in relation to each other.

ECGs may be used to diagnose a variety of heart conditions, including ventricular tachycardia. Ventricular tachycardia is a fast heart rhythm that originates in the ventricles, the lower chambers of the heart. During ventricular tachycardia, the heart may beat more than 100 times per minute, which can lead to symptoms such as dizziness, lightheadedness, and fainting. In some cases, ventricular tachycardia can be life-threatening. On an ECG, ventricular tachycardia may be identified by a wide, unusual-looking QRS complex that typically lasts longer than 120 milliseconds. In ventricular tachycardia, this may be repeated in fast sequence. It is also possible to have isolated, wide and unusual-looking QRS complexes but that may be a sign of premature ventricular contraction (PVC) rather than ventricular tachycardia. The QRS complex represents the electrical activity that occurs when the ventricles contract to pump blood out of the heart. In ventricular tachycardia, the electrical impulses that control the ventricular contractions are abnormal, leading to the characteristic changes seen on the ECG.

In addition to providing a visual representation of the heart's electrical activity, ECGs can also help to identify the underlying cause of abnormal contractions, e.g., ventricular tachycardia, such as an underlying heart disease or electrolyte imbalance. Treatment for abnormal contractions like ventricular tachycardia may include medications, implantable devices such as pacemakers or defibrillators, or catheter ablation. Catheter ablation is a minimally invasive procedure in which a thin, flexible tube (catheter) is inserted into a blood vessel and guided to the heart. The catheter delivers radiofrequency energy or extreme cold to small areas of heart tissue that are causing abnormal electrical signals, creating small scars that interrupt the abnormal electrical activities responsible for abnormal contractions.

Electrocardiography plays an important role in guiding catheter ablation procedures, such as those used to treat ventricular tachycardia. During the procedure, the ECG can be monitored continuously to help identify the location of the abnormal electrical signals causing the ventricular tachycardia. By analyzing the ECG pattern, the exact location of the arrhythmia can be pinpointed, and the catheter can be guided to that area of the heart. Once the catheter is in the correct location, the ECG can be used to confirm that the abnormal electrical signals have been successfully interrupted by the ablation procedure.

However, precise determination of the location of the arrhythmia before performing the ablation procedure, i.e., a non-invasive technique without inserting any catheter, may improve success of treatment and may reduce risks during the ablation procedure, e.g., by improved guidance of the catheter.

WO 2015/153832 A discloses a method where patient-specific cardiac electrical properties are estimated by simulating cardiac electrophysiology over time in the patient-specific anatomical heart model using a computational cardiac electrophysiology model and adjusting cardiac electrical parameters based on the simulation results and the non-invasive electrocardiography measurements.

WO 2019/145098 discloses a method for generating an EP map for at least a part of the heart from a three-dimensional surface of the patient, heart segmentation, and measured electric potentials at electrode locations on the patient. The image processor performs a sensitivity analysis using the EP map and outputs a change in position for at least one of the ECG electrodes.

The Background section of this document is provided to place embodiments of the present invention in technological and operational context, to assist those of skill in the art in understanding their scope and utility. Approaches described in the Background section could be pursued but are not necessarily approaches that have been previously conceived or pursued. Unless explicitly identified as such, no statement herein is admitted being prior art merely by its inclusion in the Background section.

### SUMMARY OF THE INVENTION

There is a need for improved techniques for determining the location of a cardiac activation area. In particular, there is a need for improved non-invasive techniques which enable determining the location of a cardiac activation area based on electrocardiography.

This object is achieved by the features of the independent patent claims. The features of the dependent patent claims define embodiments.

The following is a simplified summary of the disclosure for the purpose of providing a basic understanding to those skilled in the art. This summary is not a comprehensive overview of the disclosure and is not intended to identify key/critical elements of embodiments of the invention or to define the scope of the invention. The sole purpose of this summary is the presentation of some of the concepts disclosed herein in a simplified form as a prelude to the more detailed description that will be presented later.

Various techniques in connection with determining a location of a cardiac activation area based on electrocardiography are described below

A method for determining a cardiac activation area of a heart of a patient based on electrocardiography, ECG, comprises obtaining image data of the heart of the patient as well as obtaining image data of a torso of the patient. Furthermore, according to the method, positions of electrodes at the torso are obtained. The electrodes may include skin electrodes that can be applied non-invasively. An ECG signal is obtained. The ECG signal, hereafter referred to as the "measured ECG signal", is measured on the patient using the electrodes in combination with for example an ECG apparatus. Based on the image data of the heart, the image data of the torso, the positions of the electrodes, and the measured ECG signal, a model is parameterized. The model is configured for estimating electrical potentials on the skin of the torso of the patient depending on cardiac activity. Multiple simulated ECG signals for multiple activation patterns are determined using the model. The cardiac activation area of the heart is determined based on comparing the multiple simulated ECG signals with the measured ECG signal.

An ECG signal may comprise a 12-lead ECG. This may apply to the measured ECG signal as well as to each simulated ECG. In a 12-lead ECG, ten electrodes may be placed at the patient. The overall magnitude of the heart's electrical potential is then measured from twelve different angles ("leads") and is recorded over a period of time (usually ten seconds). In this way, the overall magnitude and direction of the heart's electrical depolarization is captured at each moment throughout the cardiac cycle.

According to various examples, a system for determining a cardiac activation area of a heart of a patient based on ECG is provided. The system comprises at least one interface and a control circuit. The system may comprise a computer system, e.g., a personal computer or server, including one or more processors as the control circuit, e.g., one or more central processing units (CPUs) or graphics processing units (GPUs).

The at least one interface is configured to obtain image data of the heart of the patient, obtain image data of a torso of the patient, obtain positions of electrodes at the torso, and obtain a measured ECG signal measured at the patient using the electrodes. The at least one interface may include one or more interfaces for data communication with imaging devices, for example cameras, computer tomography devices, sonography devices, angiography devices, x-ray devices, or any other medical or non-medical imaging device for capturing 2D or 3D images. Positions of the electrodes at the torso may be obtained based on image data of the torso captured by a stereo camera, or based on any other 3D scanning technologies, including e.g., optical, acoustic, laser, radar, or thermal scanning techniques. Positions of the electrodes at the torso may be obtained based on computer tomographic images. Positions of the electrodes at the torso may be obtained based on user input. The at least one interface may include an interface for data communication with an electrocardiograph providing for example a 12-lead ECG. The at least one interface may include an interface for communication with a database from which previously captured image data, positioning data and measured ECG signal data can be retrieved. In general, the at least one interface may include any type of interface for data communication, for example via a Local Area Network (LAN), Wireless LAN (WLAN), Bluetooth (BT), Universal Serial Bus (USB) and the like.

The control circuit is configured to parameterize a model for estimating electrical potentials on the skin of the torso of the patient depending on cardiac activity. In particular, the model is parameterized based on the image data of the heart, the image data of the torso, the positions of the electrodes, and the measured ECG signal. By use of the parameterized model, the control circuit determines multiple simulated ECG signals for multiple activation patterns and determines the cardiac activation area of the heart based on comparing the multiple simulated ECG signals with the measured ECG signal.

For example, a measured 12-lead ECG, captured 3D medical images of the heart and the torso, and information on positions of the ECG electrodes on the torso may be input as input parameters into the system, i.e., made available to the control circuit, which provides a parameterizable generic model of a human's torso and heart. Parameters of the model relating to the location and orientation of the heart and the torso may be adapted based on the corresponding images. Based on the heart and torso anatomy, the system estimates the electrical potential on the torso and adapts parameters of the model such that similarity with the input 12-lead ECG is maximized. To do so, the system produces a simulated ECG signal, e.g., by estimating the electrical potential on the torso in the corresponding ECG electrodes positions based on the model. To estimate the electrical potential on the torso at the corresponding ECG electrode positions in the model, a sinus rhythm with normal activation of the myocardium may be assumed. The model may include further parameters which may be adapted such that the similarity with the input 12-lead ECG can be maximized. For example, the conduction velocity and/or electrical diffusivity of cardiac tissue may be varied until a QRS complex of the simulated ECG signals corresponds to a QRS complex of the measured ECG signal. In further examples, the electrical conductivity of torso tissue may be varied until signal amplitudes of at least some of the simulated ECG signals match to signal amplitudes of the corresponding measured ECG signal.

Furthermore, parameterizing of the model based on the generic model may comprise varying an orientation of the heart with respect to the torso and/or varying a placement of the heart with respect to the torso for optimizing a match between at least some of the simulated ECG signals with the corresponding measured ECG signal.

The system may then estimate the electrical potential on the torso that corresponds to a variety of different activation patterns of the heart. The system may compare the simulated 12-leads ECG signals corresponding to the variety of different activation patterns with the measured 12-leads ECG signal and may determine which one of the simulated 12-leads ECG signals has the best matching to the measured 12-leads ECG signal. The activation pattern of best matching simulated 12-leads ECG signal may be used to determine the cardiac activation area of the heart.

In various examples, the system may further determine whether ECG signal variations of the simulated 12-leads ECG signals are sufficient to discriminate the underlying cardiac activation patterns from each other. If the simulated 12-leads ECG signals are not sufficient to distinguish cardiac activation patterns, i.e., two or more of the simulated 12-leads ECG signals are similar although they result from different cardiac activation patterns, the positions of the electrodes may be varied in the model and further simulated 12-leads ECG signals may be estimated for the variety of different activation patterns. This may be repeated until positions for the electrodes are found that result in sufficiently distinguishing simulated 12-leads ECG signals for the variety of different activation patterns. The thus found positions for the electrodes may be output and the positions of the electrodes at the torso may be adapted accordingly. Then, the parameterization may be repeated with the new electrode positions and a correspondingly measured ECG signal.

A computer program or a computer program product or a computer-readable storage medium comprises electronically readable control information, e.g., program code, which can be loaded and executed by a control circuit, e.g., a processor. When the control circuit executes the program code, a method for determining a cardiac activation area of a heart of a patient based on electrocardiography is implemented. The method comprises obtaining image data of the heart of the patient, obtaining image data of a torso of the patient, obtaining positions of electrodes at the torso, obtaining a measured ECG signal measured at the patient using the electrodes, and parameterizing of a model for estimating electrical potentials on the skin of the torso of the patient depending on cardiac activity. The model is parameterized based on the image data of the heart, the image data of the torso, the positions of the electrodes, and the measured ECG signal. The method further comprises determining multiple simulated ECG signals for multiple activation patterns using the model and determining the cardiac activation area of the heart based on comparing the multiple simulated ECG signals with the measured ECG signal.

The features set out above and features that are described below may be used not only in the corresponding combinations explicitly set out, but also in further combinations or in isolation, without departing from the scope of protection of the present invention.

### BRIEF DESCRIPTION OF THE DRAWINGS

The present invention will now be described more fully hereinafter with reference to the accompanying drawings, in which embodiments of the invention are shown. However, this invention should not be construed as limited to the embodiments set forth herein. Rather, these embodiments are provided so that this disclosure will be thorough and complete, and will fully convey the scope of the invention to those skilled in the art. Like numbers refer to like elements throughout.
FIG. 1 shows schematically a system for determining a cardiac activation area of a heart of a patient according to an embodiment.
FIG. 2 shows method steps of a method for determining a cardiac activation area of a heart of a patient according to an embodiment.

### DETAILED DESCRIPTION OF THE INVENTION

The properties, features and advantages of this invention described above and the way in which they are achieved will become clearer and more clearly understood in association with the following description of the exemplary embodiments which are explained in greater detail in connection with the drawings.

For simplicity and illustrative purposes, the present invention is described by referring mainly to an exemplary embodiment thereof. In the following description, numerous specific details are set forth in order to provide a thorough understanding of the present invention. However, it will be readily apparent to one of ordinary skill in the art that the present invention may be practiced without limitation to these specific details. In this description, well known methods and structures have not been described in detail so as not to unnecessarily obscure the present invention.

Various examples of the present disclosure relate to systems and methods for determining a cardiac activation area of a heart of a patient based on electrocardiography. The disclosed systems and methods allow a non-invasive identification of the cardiac activation area. Non-invasive identification of a cardiac activation area may be used for identification of a target site for ablation as part of for example ventricular tachycardia (VT) treatment. Generally, as will be described below, the target site or area may be approximately estimated by analyzing signal patterns of a 12-lead electrocardiogram (ECG). It has been found that the position of ECG electrodes affects the signal features especially in the precordial region. ECG electrodes are positioned following general rules based on population-average anatomic models. Furthermore, it has been found that there may be an uncertainty on the patient-specific anatomy, for example mutual position and size of the heart and the torso, and on the position of the ECG electrodes with respect to the patient's heart. This may limit precision of determining the target size or area. Additionally, there is intrinsic ambiguity in the ECG signal, for example when the early activation is localized in basal or septal areas. The tissue abnormality that is responsible for VT re-entry could be localized in different anatomical structures occupying the same area, for example left or right ventricle. In this case, the ECG may contain features that are not clearly discriminative of the different scenarios. However, it may be important to know exactly what anatomical structure hosts the target for ablation, since this affects planning and execution of the ablation procedure.

Generally, in view of the above, personalized computational electrophysiological models may be applied to estimate areas of early activation from measured 12-lead ECGs. For example, 3D imaging showing the patient's heart may be captured. 12-lead ECG measurements in sinus rhythm and/or during VT may be captured. Based on the 3D imaging of the heart and the 12-lead ECG, a computational model of electrophysiology may be built. The model may be capable of producing simulated 12-lead ECG signals. The area of early activation of the patient's heart may be inferred from the area of early activation computed by the computational model, when the simulated and measured ECG signals are matching.

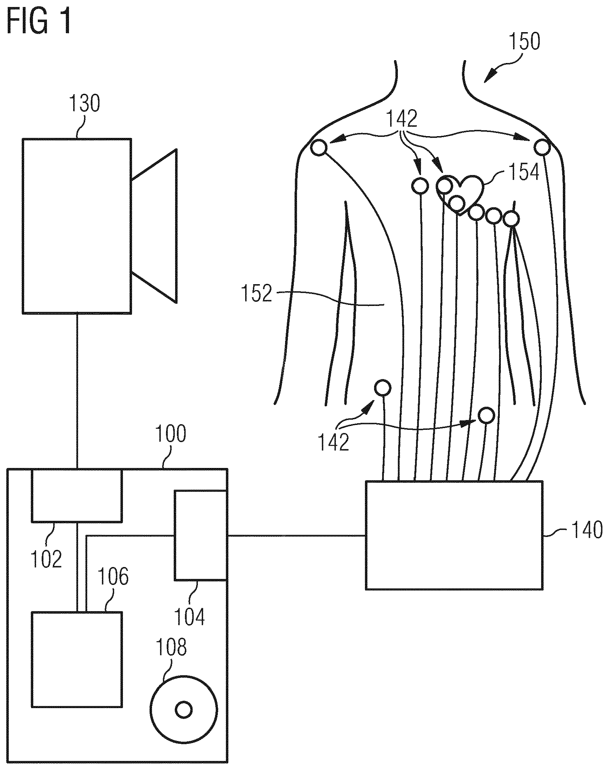

As such, an exemplary system 100 for determining a cardiac activation area in connection with a patient 150 is shown in FIG. 1.

The system 100 comprises one or more interfaces 102 for obtaining image data of the heart 154 and the torso 152 of the patient 150. The interface(s) 102 may be directly coupled to corresponding capturing device(s) 130. The image data may include 3D images. The capturing device 130 may include a magnetic resonance or computed tomography device, a sonography device, an x-ray device, or a camera device, for example a 3D camera system. Imaging techniques may include 3D echocardiography imaging, cardiac magnetic resonance (CMR) imaging, and cardiac-gated computed tomography (Cardiac CT) imaging. Cardiac CT examinations may include acquisitions with sufficient field of view that the torso 152 can be reconstructed via segmentation. In case of CMR, specific "localizer" acquisitions may be performed which depict the chest area so that the torso can be segmented. Additionally, positions of electrodes 142 of an ECG apparatus 140 may be obtained via the interface 102. The electrodes 142 may include skin electrodes attached to the torso 152, for example by use of adhesives or suction. The electrodes 142 may be visible in computed tomography (CT) examinations since they may include metallic components. In magnetic resonance imaging (MRI), skin markers at the location of the electrodes may be used. As a result, the system 100 may obtain via the one or more interfaces 102 image data of the heart 154, image data of the torso 152, and information on the positions of the ECG electrodes 142 at the torso 152.

The system 100 may comprise a further interface 104 for obtaining an ECG signal from the ECG apparatus 140. The ECG apparatus 140 measures the ECG signal at the patient 150 using the electrodes 142. In particular, the ECG apparatus 140 may provide to the interface 104 a measured 12-lead ECG signal based on the measurements performed at the patient 150 using the electrodes 142. Although the interface 104 is shown as a separate interface, it may be integrally formed with the interface(s) 102, i.e., the interface(s) 102 may provide a coupling to the capturing device 130 and the ECG apparatus 140. Generally, the information from the capturing device 130 and the ECG apparatus 140 can be received directly from these devices as livestreams or can be retrieved from a database in which the information has been stored previously. The interfaces 102, 104 may include for example an interface to a local area network (LAN), an interface to a wireless LAN (WLAN) or any other standardized or custom specific data communication interface.

The system 100 comprises a control circuit 106 coupled to the interfaces 102, 104 such that the control circuit 106 can process the information obtained via the interfaces 102, 104. The control circuit 106 may comprise a processor, for example a digital general purpose central processing unit (CPU) or graphics processing unit (GPU) along with memory for storing data and program code, for example random access memory (RAM), read only memory (ROM, and/or flash memory), and input/output (I/O) units for inputting and outputting information from/to a user interface and a data carrier 108, for example a hard disk, a CD-ROM and/or flash memory. As such, the system 100 may comprise a computer, for example a personal computer, a notebook, or a server. The data carrier 108 may comprise electronically readable control information stored thereon which can be loaded into the memory of the control unit 106 and which is configured to control the control unit 106 to process the information obtained via the interfaces 102, 104 as will be described below.

Based on a model, for example a generic electrophysiology model of a human's torso and heart, and the information on the obtained heart and torso anatomy, the control unit 106 estimates the electrical potential on the torso which maximizes the similarity with the measured 12-lead ECG. According to the invention, the control unit 106 parameterizes the generic electrophysiology model based on the image data of the heart 154, the image data of the torso 152, the positions of the electrodes 142, and the measured 12-lead ECG. Parameters of the model which may be varied for parameterizing the model may include properties of the heart and the torso, in particular electrical properties of the tissue of the heart and the torso, as well as the position and/or orientation of the heart 154 within the torso 152. For example, the control circuit 106 may produce simulated ECG signals by "sampling" the electrical potentials on torso at the corresponding ECG electrode positions using the parameterized model. "Sampling" the electrical potentials on the torso using the parameterized model means that the control circuit 106 calculates, using the model, electrical potentials for those locations at the torso where the electrodes are arranged at the patient's torso. A corresponding simulated 12-lead ECG signal can then be derived from the electrical potentials at those locations. It is clear that, upon applying an activation pattern to the heart, at each electrode location a sequence of varying electrical potentials is sampled which indicates the voltage curve at that electrode location. Based on these simulated voltage curves, the simulated 12-lead ECG signal is determined. Parameterizing the model may be performed by varying the above parameters and comparing the corresponding simulated ECG signal with the measured ECG signal. The parameters are varied such that the matching between the simulated ECG signal and the measured ECG signal is maximized. During parameterization of the model, a "normal" activation pattern may be applied to the heart in the model, for example an activation pattern where the heartbeat activation starts in the sinus node of the heart and no additional ventricular activation occurs. However, depending on the measured ECG signal, another activation pattern may be applied during parameterization of the model that appear to be more appropriate, for example an activation pattern where the activation of the heartbeat has its origin in a ventricle, or an early activation occurs in a septal endocardial point.

As noted above, the voltage curves at the electrode locations depend on the type of activation pattern applied to the heart in the model. Therefore, when the model is parameterized, the control unit 106 may determine multiple simulated 12-lead ECGs for multiple activation patterns using the parameterized model. The multiple activation patterns may include a normal activation of the myocardium starting in the septal endocardial, an early activation in a predefined set of septal endocardial points, and an early activation in a predefined set of non-septal endocardial points. The thus derived multiple simulated ECG signals for the multiple activation patterns may be compared by the control circuit 106 with the measured ECG signal and the activation pattern corresponding to the best matching simulated ECG signal may be assumed to correspond to the activation pattern acting in the patient's heart 152. Based on this activation pattern, a catheter ablation procedure may be planned.

Additionally, based on the plurality of simulated ECG signals, the control circuit 106 may determine whether the simulated ECG signals are sufficiently different from each other such that one of the activation patterns may be identified with sufficient certainty based on the simulated ECG signals. For example, if some of the simulated ECG signals are similar although they are based on significantly different activation patterns, it may be determined that the simulated ECG signals are not sufficiently different from each other for identifying the underlying activation pattern. In this case, the position of the electrodes may be varied in the parameterized model and further simulated ECG signals may be determined by applying the multiple activation patterns again. The control circuit 106 may then determine whether the further simulated ECG signals are sufficiently different from each other such that one of the activation patterns can be identified with sufficient certainty. This may be repeated until the simulated ECG signals sufficiently differ from each other. The thus determined positioning of the electrodes may be output to a user of the system 100 as a proposal to rearrange the electrodes 142 at the patient 150 to achieve more reliable information for ablation planning.

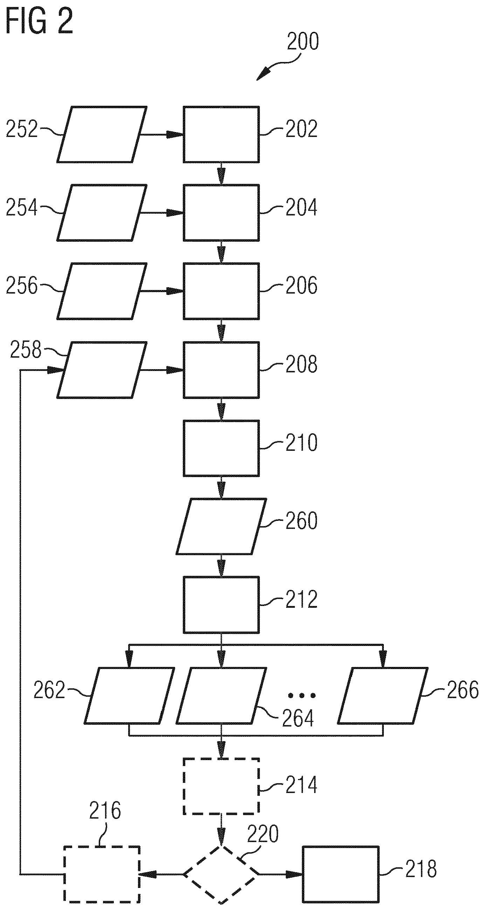

In the following, the above-described techniques will be described in more detail with reference to a method 200 shown as a flowchart in FIG. 2. The method steps of the method 200 may be performed by the control circuit 106 and may be defined in program code stored on data carrier 108.

As explained above, in step 202, image data 252 of the heart 154 of the patient 150 is obtained. In step 204, image data 254 of the torso 152 of the patient 150 is obtained. In step 206, a measured ECG signal 256 is obtained. The measured ECG signal 256 may be output by the ECG apparatus 140 upon measurements at the patient 150 using the electrodes 142. In step 208, positions 258 of the electrodes 142 at the torso 152 are obtained. A generic model of a human torso and heart is provided in the control circuit 106 and is parameterized in step 210 based on the image data 252 of the heart 154, the image data 254 of the torso 152, the positions 258 of the electrodes 142, and the measured ECG signal 256. The model is capable to simulate or estimate electrical potentials on the skin of the torso depending on cardiac activity. A plurality of activation patterns may be provided in the control circuit 106 which may be used in connection with the model for activating and simulating cardiac activity. I.e. upon selecting an activation pattern, the model simulates electrical potentials on the skin of the torso, for example at a plurality of predefined points on the skin of the torso.

Parameterization of the generic model will be described in more detail in the following.

The generic model may be capable of producing ECG signals under varying configurations representing for example modified size and geometry of the heart, of the torso, the mutual arrangement in space, and positions of the ECG electrodes. Furthermore, physical properties may be considered and varied, in particular electrical properties. For example, a computational model of electrophysiology capable of estimating electrical potentials on the torso may be utilized. Methods to create such computational models are well known in the art. Additionally or as an alternative, a data-driven model of electrical potentials on the torso may be utilized.

In detail, given the input information (anatomical images of the heart and torso, ECG electrodes position, and a corresponding 12-lead ECG signal), a personalized computational model of electrophysiology can be defined by a discrete set of spatial locations, each associated with a set of (input) features, and for which variables of interests can be computed. For example, the generic model may comprise a finite elements representation of the heart and the torso. A discrete set of points may be organized in a tetrahedral mesh representing the anatomy of the heart, and in a triangular mesh representing the anatomy of the torso. However, finite elements having other shapes may be utilized, e.g., tetrahedral meshes for the heart as well as for the torso. Each discrete point may be associated with, for example, a corresponding estimated electrical potential, a conduction velocity of corresponding tissue, and/or an electrical diffusivity of corresponding tissue.

As part of the personalization process, i.e., the parameterization of the model, the following may be carried out. Optimal physical properties of the cardiac tissue and the torso tissue may be estimated. For instance, the conduction velocity of the cardiac tissue can be iteratively varied until the generated/simulated ECG signal exhibits a QRS complex with the same duration as the measured ECG signal. Also, the conductivity of the torso can be modified to optimize the match of the simulated ECG signal amplitude in each lead compared to the measured ECG. The optimal orientation of the heart with respect to the torso may be estimated. This accounts for uncertainty in the image segmentation process. For instance, multiple options of arrangement in 3D space can be tested and simulated. The heart can be placed in multiple locations within the torso, and with multiple rotations, and the position and rotation that leads to the optimal match of the generated/simulated ECG signal with the measured ECG signal is selected as the optimal position and rotation.

The personalized parameterized virtual ECG model may include the optimal values of conduction velocities in the heart, and the optimal arrangement of heart and torso in space, which maximizes the match of the generated/simulated ECG with the measured ECG. One possible way to quantify the match is by computing the beat-by-beat correlation of each of the generated/simulated ECG lead signals with the corresponding measured ECG lead signals.

As a result, a parameterized model 260 is created.

Based on the parameterized model 260, in step 212 multiple simulated ECG signals 262-266 for multiple activation patterns may be determined. For example, corresponding simulated ECG signals 262-266 can be determined based on the electrical potentials on the skin of the torso. For example, a plurality of different activation patterns may be provided. The activation patterns may be applied successively to the parameterized model 260 and for each applied activation pattern corresponding electrical potentials on the skin of the torso are simulated. Based on the simulated electrical potentials a corresponding simulated ECG signal 262-266 may be determined.

In more detail, the parameterized ECG model may be used to produce multiple variations of 12-lead ECG signals, corresponding to different activation patterns. For example, sinus rhythm ECG signals may have been originally provided as an input to the model, so the personalized parameterized ECG model is optimized to match sinus rhythm ECG signals. In sinus rhythm, normal activation of the myocardium generally starts in the septal endocardium, then proceeds to activate the left ventricular and right ventricular endocardia, and finally proceeds to the free walls and epicardia. The precise activation sequence may be unknown since it cannot be directly inferred from surface ECG measurements. Nonetheless, multiple different hypotheses can be formulated and tested with the parameterized ECG model. For example, early activation in a sparse set of septal endocardial points (either randomly selected or according to predefined data, e.g., from literature), i.e., in the ventricular endocardium, may be simulated. In further examples, early activation in a sparse set of septal and non-septal endocardial points (either randomly selected or according to predefined data, e.g., from literature), i.e., in the ventricular epicardium, may be simulated.

The parameterized model may then be used to produce a library of simulated ECG signals 262-266, each corresponding to a different activation pattern. In step 218, one of the simulated ECG signals in the library is selected as the best matching to the measured ECG signal. For example, the matching can be quantified by calculating the average or minimum correlation between all the simulated ECG leads and the corresponding measured ECG leads. The activation pattern of the best matching simulated ECG signal may be considered to represent the activation pattern that led to the measured ECG signal. Based on this activation pattern of the best matching simulated ECG signal, an early activation area of the heart 154 of the patient 150 may be determined.

In various examples, the library includes ECG signals generated by varying all variables in the ECG model (size and position of the heart and the torso, physical properties of the cardiac tissue). A simulated ECG signal may be identified as the best match to the measured ECG signal in two steps. First, a subset of simulated ECG signals is extracted from the library based on the similarity between the size and position of the heart and torso extracted from the input 3D medical images and used for the generation of the signals in the library. Then, the best signal from the subset is selected based on a matching metric described above.

Before or in addition to determining the best match between the simulated ECG signal and the cardiac activation area, the method may be further improved by changing the positioning of the electrodes.

To accomplish this, in step 214, an average matching metric dependent on the simulated ECG signals 262-266 and/or the measured ECG signal 256 is determined, and depending on the average matching metric, the position of the electrodes in the model may be varied and further multiple simulated ECG signals may be simulated for the various activation patterns.

Generally, the library of simulated ECG signals contains multiple signals which can be compared with each other. For example, the comparison may be performed by quantifying the match as described above to identify the best matching ECG signal. The matching between each pair of simulated ECG signals in the library may be computed and an average matching metric may be computed for the entire library. Additionally, or as an alternative, a matching metric may be computed between the measured ECG signal and any simulated ECG signal from the library. Also in this case, an average matching metric can be computed for the entire library. In further examples, the average matching metric may be computed only for the k nearest neighbors, i.e., the k simulated ECG signals in the ECG library with the best match to the measured ECG signal.

If the matching metric for the library exceeds a set threshold, this means that the multiple simulated ECG signals match very well with each other though they are based on different activation patterns. Consequently, different activation patterns may not be identified with high confidence based on the ECG signals because they are not significantly different from each other. Similarly, if the number k of nearest neighbors that result in a matching metric that exceeds a set threshold is greater than a given value kₘₐₓ, this means that these k simulated ECG signals do not significantly differ from each other even though they are based on different activation patterns. If such matching is determined in step 220, better ECG electrode positions may be determined in step 216. For example, each ECG electrode position in the model may be displaced on the torso surface by a fixed distance (e.g., randomly selected) in one direction (e.g., randomly selected). For each new set of ECG electrode positions, the method may be repeated starting at step 208, with the new set of ECG electrode positions replacing the positions 258 obtained from the image data. It should be noted that the "real world" conditions may remain unchanged, at least for the time being. Thus, further multiple simulated ECG signals are computed with the same multiple activation patterns as in the previous pass (steps 208 to 212). A new library of simulated ECG signals 262-266 is populated. The matching metric for each pair of simulated ECG signals in the new library is computed (step 214), and the average matching metric for the library is derived. If the matching metric for the library exceeds the set threshold (step 220), the new ECG electrodes position is discarded and a further repositioning of the electrodes in the model may be performed (step 216). Varying the positions of the electrodes in step 216 may comprise for example, varying the position of at least one of the electrodes, removing one of the electrodes, and/or including one or more additional electrodes at additional positions.

Generally, new ECG electrode positions may be generated in the model until the matching metric of the library is below the set threshold, for at least one of the ECG electrodes positions. Additionally, or alternatively, new ECG electrode positions may be generated until a certain number of proposals have been generated, irrespective of the corresponding library matching metric. If at least one set of ECG electrode positions is found, for which the corresponding library matching metric is below the set threshold, then that set of ECG electrode positions is output to the user. If no new set of ECG electrode positions is found for which the library matching metric is below the set threshold, then no alternative set of ECG electrode positions is output to the user, and the ECG electrode positioning as captured at the torso may be maintained. In some examples, the sets of ECG electrode positions for which the library matching metric is minimal across all proposed ECG electrodes positions may be output to the user.

The user may then reposition the electrodes at the torso 152 of the patient 150 and the method may then be repeated, at least starting with obtaining the positions 258 of the electrodes at the torso 152 in step 208.

As mentioned above, the system may propose not only alternative positions of the existing ECG electrodes, but also addition or removal of ECG electrodes. This can be achieved for instance as follows.

All simulated ECG signals in the library corresponding to the multiple activation patterns may be considered. For each simulated ECG signal, the computed electrical potentials in each point of the torso surface are considered. For each torso point, a lead is defined where the torso point acts as the positive pole, while the opposing pole is the average potential on the torso, e.g., determined based on an average potential of the computed electrical potentials. For each lead, one time-invariant feature is extracted, for example the QRS time integral.

As a result, for each simulated ECG signal in the library a vector of features (e.g., the QRS time integral) is determined, with the number of elements equal to the number of points on the torso. Next, a Principal Component Analysis (PCA) is applied on the vectors, for example a Karhunen-Loeve transform, to approximate the space of vectors in the library as the linear combination of a small set of generator vectors (e.g., 3). For each generator vector, two or more multiples (e.g., with coefficients +1 and -1) are considered. For each multiple of each generator vector, the torso leads with the largest feature (e.g., QRS time integral) are localized, and marked as candidate location for an ECG electrode. Depending on the number of generator vectors selected in the above process, a number of ECG electrodes can be recommended that can be larger or smaller than the original number. Also, their optimal location on the torso can be recommended.

Once a new library is created, based on the new ECG electrode positions, the simulated ECG signal in the library that best matches the measured ECG signal is identified. If the number of electrodes has been modified, then the matching metric may be defined based on the common leads in the measured ECG and in the new simulated ECG signals.

To sum up, the above-described techniques allow to quantify whether the ECG electrodes as positioned on the torso of a patient can allow the proper discrimination between different activation patterns. Additionally, they recommend updated positions of the ECG electrodes for the specific goal of optimizing the discrimination of different activation patterns.

A typical clinical workflow using the above techniques may comprise the following.

The electrodes 142 of the ECG apparatus 114 are attached to the patient 150 in a manner known in the art. For example, ten electrodes may be placed on the patient 150 to record a 12-lead ECG over a period of time, such as 10 seconds, as a measured ECG signal. 3D images of the torso 152 and heart 154 of the patient 150 may be acquired using, for example, magnetic resonance or computed tomography, sonography, x-ray, or optical techniques. For example, 3D echocardiography, cardiac magnetic resonance, or cardiac computed tomography may be used. In addition, the positioning of the electrodes 142 on the patient 150, particularly on the torso 152 and in spatial relation to the heart 154, may be obtained using the above acquisition techniques. The image data of the heart and the image data of the torso, as well as the positions of the electrodes on the torso, are provided to the system 100. Electrocardiography is performed and an ECG is generated. The measured ECG signal is provided to the system 100.

The information listed above may be provided to the system 100 in real time or near real time or may be recorded and provided to the system 100 "off-line".

The system 100 performs the method described above in connection with FIG. 2. As described above, the system 100 may determine a better arrangement for the electrodes 142 and may provide a corresponding recommendation to an operator of the system 100. Based on the recommendation, the operator may rearrange the electrodes 142 as determined by the system 100, may include additional electrodes, or may remove some of the electrodes 142. With the new arrangement of the electrodes, an electrocardiography may again be performed, producing a further ECG signal. The further ECG signal is provided to the system 100. The system may recommend a further rearrangement of the electrodes and the process may be repeated. However, if the process converges, further rearrangement of the electrodes may not be recommended by the system 100 or may be aborted by the operator.

As a result, an accurate model of the patient's heart within the patient's torso is achieved, and the system may output an activation pattern of the heart 154 that produces an ECG signal which corresponds to the ECG signal measured with the electrodes 142 in the appropriate positions determined above.

The activation pattern may be output by the system 100. For example, the system 100 may output at which point or at which points of the heart 154 a heartbeat is activated. For example, the activation pattern may indicate a ventricular activation. Based on the activation pattern, a physician may determine how to treat an abnormal behavior of the heart. For example, the physician may recommend an ablation. Since each activation pattern is based on the specific arrangement of one or more activation points in the model of the heart 154, a physician may determine an ablation strategy to deactivate such points.

Also during ablation, the model of the heart 154 within the torso 152 may be used to monitor the response of the heart 154 to ablation.

Generally, all terms used herein are to be interpreted according to their ordinary meaning in the relevant technical field, unless a different meaning is clearly given and/or is implied by the context in which they are used. All references to a/an/the element, apparatus, component, means, step, etc. are to be interpreted openly as referring to at least one instance of the element, apparatus, component, means, step, etc., unless explicitly stated otherwise. The steps of any method disclosed herein need not be performed in the exact order disclosed, unless a step is explicitly described as following or preceding another step and/or where it is implicit that a step must follow or precede another step. Any feature of any of the embodiments disclosed herein may be applied to any other embodiment, where appropriate. Likewise, any advantage of any of the embodiments may apply to any other embodiment and vice versa. Other objectives, features and advantages of the enclosed embodiments will be apparent from the description. As used herein, the term "configured to" means set up, organized, adapted, or arranged to operate in a particular way; the term is synonymous with "designed to". As used herein, the term "substantially" means nearly or essentially, but not necessarily completely; the term encompasses and accounts for mechanical or component value tolerances, measurement errors, random variation, and similar sources of inaccuracy.

The present invention may, of course, be carried out in ways other than those specifically set forth herein without departing from essential characteristics of the invention. The present embodiments are to be considered in all respects as illustrative and not restrictive, and all modifications coming within the meaning and equivalency range of the appended claims are intended to be embraced therein.

## Claims

1. A method for determining a cardiac activation area of a heart (154) of a patient (150) based on electrocardiography, ECG, the method (200) comprising:
- obtaining (202) image data (252) of the heart (154) of the patient (150),
- obtaining (204) image data (254) of a torso (152) of the patient (150),
- obtaining (208) positions (258) of ECG electrodes (142) at the torso (152),
- obtaining (206) a measured ECG signal (256) measured at the patient (150) using the ECG electrodes (142),
- parameterizing (210) of a model (260) for estimating electrical potentials on the skin of the torso of the patient (150) depending on cardiac activity, wherein the model is parameterized based on the image data (252) of the heart (154), the image data (254) of the torso (152), the positions (258) of the ECG electrodes (142), and the measured ECG signal (256),
- determining (212) multiple simulated ECG signals (262-266) for multiple patterns of cardiac activity by sampling the electrical potentials on the torso at the corresponding ECG electrode positions using the model (260),
- determining (218) the cardiac activation area of the heart (154) based on comparing the multiple simulated ECG signals (262-266) with the measured ECG signal (256), **characterised in that** the method further comprises:
- determining (214) an average matching metric based on the simulated ECG signals (262-266) and/or the measured ECG signal (256),
- depending on the average matching metric, varying (216) a position (258) of the ECG electrodes in the model and simulating (212) further multiple simulated ECG signals (262-266) for the various patterns of cardiac activity using the model (260).

2. The method of claim 1, wherein said parameterizing (210) the model (260) is based on a generic model configured to provide ECG signals based on at least one of
- a size of the heart (154),
- a geometry of the heart (154),
- a size of the torso (152),
- a geometry of the torso (152),
- an arrangement of the heart (154) with respect to the torso (152), and
- an arrangement of the positions (258)of the electrodes (142).

3. The method of claim 2, wherein the generic model is configured to estimate electrical potentials on the torso (152) based on a computational model of electrophysiology.

4. The method of claim 2 or claim 3, wherein the generic model comprises a mesh representation of the heart (154) and/or the torso (152), wherein each mesh vertex is associated with at least one of
- a corresponding estimated electrical potential,
- a conduction velocity of corresponding tissue, and
- an electrical diffusivity of corresponding tissue.

5. The method of any one of claims 2-4, wherein parameterizing (210) of the model (260) based on the generic model comprises at least one of:
- varying a conduction velocity and/or electrical diffusivity of cardiac tissue until a QRS complex of the simulated ECG signals (262-266) corresponds to a QRS complex of the measured ECG signal (256), and
- varying the electrical conductivity of torso tissue until signal amplitudes of at least some of the simulated ECG signals (262-266) match to signal amplitudes of the corresponding measured ECG signal (256).

6. The method of any one of claims 2-5, wherein parameterizing (210) of the model (260) based on the generic model comprises at least one of:
- varying an orientation of the heart (154) with respect to the torso (152) for optimizing a match between at least some of the simulated ECG signals (262-266) with the corresponding measured ECG signal (256), and
- varying a placement of the heart (154) with respect to the torso (152) for optimizing a match between at least some of the simulated ECG signals (262-266) with the corresponding measured ECG signal (256).

7. The method of any one of claims 2-6, wherein the generic model is configured to estimate electrical potentials on the torso (152) based on a data-driven model.

8. The method of any one of the preceding claims, wherein the various patterns of cardiac activity comprise at least one of:
- a normal activation of the myocardium starting in the septal endocardium,
- early activation in a predefined set of septal endocardial points,
- early activation in a predefined set of non-septal endocardial points, and
- early activation in a predefined set of non-endocardial points.

9. The method of any one of the preceding claims, wherein the ECG signal (256, 262-266) comprises a 12-lead ECG.

10. The method of claim 1, further comprising:
- repeating the steps of determining (214) the average matching metric, varying (216) the position of the ECG electrodes, and simulating (212) further multiple ECG signals (262-266) if (220) the average matching metric exceeds a predefined threshold.

11. The method of claim 1 or claim 10, wherein determining (214) the average matching metric comprises determining the average matching metric based on matching metrics for each pair of the simulated ECG signals (262-266).

12. The method of any one of claims 1, 10 or 11, wherein determining (214) the average matching metric comprises determining the average matching metric based on matching metrics for each pair of a predefined number of the simulated ECG signals (262-266) having the best match with respect to the measured ECG signal (256).

13. The method of any one of claims 1, or 10-12, wherein the step of varying (216) the position (258) of the ECG electrodes comprises at least one of:
- varying the position of at least one of the ECG electrodes,
- removing one of the ECG electrodes, and
- including an additional ECG electrode at an additional position.

14. A system for determining a cardiac activation area of a heart (154) of a patient (150) based on electrocardiography, ECG, the system (100) comprising:
- at least one interface (102, 104) configured to
obtain (202) image data (252) of the heart (154) of the patient (150),
obtain (254) image data (254) of a torso (152) of the patient (150),
obtain (208) positions (258) of ECG electrodes (142) at the torso (152), and
obtain (206) a measured ECG signal (256) measured at the patient (150) using the ECG electrodes (142), and
- a control circuit (106) configured to
parameterize (210) a model (260) for estimating electrical potentials on the skin of the torso (152) of the patient (150) depending on cardiac activity, wherein the model (260) is parameterized based on the image data (252) of the heart (154), the image data (254) of the torso (152), the positions (258) of the ECG electrodes (142), and the measured ECG signal (256),
determine (212) multiple simulated ECG signals (262-266) for multiple patterns of cardiac activity by sampling the electrical potentials on the torso at the corresponding ECG electrode positions using the model (260),
determine (218) the cardiac activation area of the heart (154) based on comparing the multiple simulated ECG signals (262-266) with the measured ECG signal (256), **characterised in that** the control circuit is further configured to
determine (214) an average matching metric based on the simulated ECG signals (262-266) and/or the measured ECG signal (256), and
depending on the average matching metric, varying (216) a position (258) of the ECG electrodes in the model and simulating (212) further multiple simulated ECG signals (262-266) for the various patterns of cardiac activity using the model (260).

15. The system of claim 14, wherein the control circuit (106) is configured to perform the method (200) of any one of claims 1-14.

16. A computer program product comprising a program which is directly loadable into a memory of a programmable control circuit (106), comprising program means for executing all steps of the method (200) according to any one of claims 1-13 when the program is executed in the control circuit (106).

17. A data carrier comprising electronically readable control information stored thereon, which is configured to perform the method (200) according to any of claims 1-13 when the data carrier (108) is used in a control circuit (106).

## Patentansprüche

1. Verfahren zum Bestimmen eines Herzaktivierungsbereichs eines Herzens (154) eines Patienten (150) basierend auf Elektrokardiographie, EKG, wobei das Verfahren (200) Folgendes umfasst:
- Erhalten (202) von Bilddaten (252) des Herzens (154) des Patienten (150),
- Erhalten (204) von Bilddaten (254) eines Oberkörpers (152) des Patienten (150),
- Erhalten (208) von Positionen (258) von EKG-Elektroden (142) an dem Oberkörper (152),
- Erhalten (206) eines gemessenen EKG-Signals (256), das an dem Patienten (150) unter Verwendung der EKG-Elektroden (142) gemessen wird,
- Parametrisieren (210) eines Modells (260) zum Schätzen elektrischer Potentiale auf der Haut des Oberkörpers des Patienten (150) in Abhängigkeit von der Herzaktivität, wobei das Modell basierend auf den Bilddaten (252) des Herzens (154), den Bilddaten (254) des Oberkörpers (152), den Positionen (258) der EKG-Elektroden (142) und dem gemessenen EKG-Signal (256) parametrisiert wird,
- Bestimmen (212) von mehreren simulierten EKG-Signalen (262-266) für mehrere Muster der Herzaktivität durch Abtasten der elektrischen Potentiale an dem Oberkörper an den entsprechenden EKG-Elektrodenpositionen unter Verwendung des Modells (260),
- Bestimmen (218) des Herzaktivierungsbereichs des Herzens (154) basierend auf Vergleichen der mehreren simulierten EKG-Signale (262-266) mit dem gemessenen EKG-Signal (256),
**dadurch gekennzeichnet, dass** das Verfahren ferner Folgendes umfasst:
- Bestimmen (214) einer durchschnittlichen Übereinstimmungsmetrik basierend auf den simulierten EKG-Signalen (262-266) und/oder dem gemessenen EKG-Signal (256),
- Variieren (216) einer Position (258) der EKG-Elektroden in dem Modell und Simulieren (212) von weiteren mehreren simulierten EKG-Signalen (262-266) für die verschiedenen Muster der Herzaktivität unter Verwendung des Modells (260) in Abhängigkeit von der durchschnittlichen Übereinstimmungsmetrik.

2. Verfahren nach Anspruch 1, wobei das Parametrisieren (210) des Modells (260) auf einem generischen Modell basiert, das konfiguriert ist, um EKG-Signale basierend auf mindestens einem von Folgenden bereitzustellen
- einer Größe des Herzens (154),
- einer Geometrie des Herzens (154),
- einer Größe des Oberkörpers (152),
- einer Geometrie des Oberkörpers (152),
- einer Anordnung des Herzens (154) in Bezug auf den Oberkörper (152) und
- einer Anordnung der Positionen (258) der Elektroden (142).

3. Verfahren nach Anspruch 2, wobei das generische Modell konfiguriert ist, um elektrische Potentiale an dem Oberkörper (152) basierend auf einem Berechnungsmodell der Elektrophysiologie zu schätzen.

4. Verfahren nach Anspruch 2 oder 3, wobei das generische Modell eine Netzdarstellung des Herzens (154) und/oder des Oberkörpers (152) umfasst, wobei jeder Netzscheitelpunkt mit mindestens einem von Folgenden verknüpft ist:
- einem entsprechenden geschätzten elektrischen Potential,
- einer Leitungsgeschwindigkeit des entsprechenden Gewebes und
- einer elektrischen Diffusionsfähigkeit des entsprechenden Gewebes.

5. Verfahren nach einem der Ansprüche 2 bis 4, wobei das Parametrisieren (210) des Modells (260) basierend auf dem generischen Modells mindestens eines von Folgenden umfasst:
- Variieren einer Leitungsgeschwindigkeit und/oder elektrischen Diffusionsfähigkeit des Herzgewebes, bis ein QRS-Komplex der simulierten EKG-Signale (262-266) einem QRS-Komplex des gemessenen EKG-Signals (256) entspricht, und
- Variieren der elektrischen Leitfähigkeit des Oberkörpergewebes, bis Signalamplituden mindestens einiger der simulierten EKG-Signale (262-266) mit Signalamplituden des entsprechenden gemessenen EKG-Signals (256) übereinstimmen.

6. Verfahren nach einem der Ansprüche 2 bis 5, wobei das Parametrisieren (210) des Modells (260) basierend auf dem generischen Modells mindestens eines von Folgenden umfasst:
- Variieren einer Ausrichtung des Herzens (154) in Bezug auf den Oberkörper (152) zum Optimieren einer Übereinstimmung zwischen mindestens einigen der simulierten EKG-Signale (262-266) mit dem entsprechenden gemessenen EKG-Signal (256) und
- Variieren einer Platzierung des Herzens (154) in Bezug auf den Oberkörper (152) zum Optimieren einer Übereinstimmung zwischen mindestens einigen der simulierten EKG-Signale (262-266) mit dem entsprechenden gemessenen EKG-Signal (256).

7. Verfahren nach einem der Ansprüche 2 bis 6, wobei das generische Modell konfiguriert ist, um elektrische Potentiale an dem Oberkörper (152) basierend auf einem datengesteuerten Modell zu schätzen.

8. Verfahren nach einem der vorhergehenden Ansprüche, wobei die verschiedenen Muster der Herzaktivität mindestens eines von Folgenden umfassen:
- eine normale Aktivierung des Myokards, die im Septumendokard beginnt,
- frühe Aktivierung in einer vordefinierten Gruppe von septalen endokardialen Punkten,
- frühe Aktivierung in einer vordefinierten Gruppe von nichtseptalen endokardialen Punkten und
- frühe Aktivierung in einer vordefinierten Gruppe von nichtendokardialen Punkten.

9. Verfahren nach einem der vorhergehenden Ansprüche, wobei das EKG-Signal (256, 262-266) ein 12-Kanal-EKG umfasst.

10. Verfahren nach Anspruch 1, ferner umfassend:
- Wiederholen der Schritte des Bestimmens (214) der durchschnittlichen Übereinstimmungsmetrik, des Variierens (216) der Position der EKG-Elektroden und des Simulierens (212) von weiteren mehreren EKG-Signalen (262-266), wenn (220) die durchschnittliche Übereinstimmungsmetrik einen vordefinierten Schwellenwert überschreitet.

11. Verfahren nach Anspruch 1 oder Anspruch 10, wobei das Bestimmen (214) der durchschnittlichen Übereinstimmungsmetrik Bestimmen der durchschnittlichen Übereinstimmungsmetrik basierend auf Übereinstimmungsmetriken für jedes Paar der simulierten EKG-Signale (262-266) umfasst.

12. Verfahren nach einem der Ansprüche 1, 10 oder 11, wobei das Bestimmen (214) der durchschnittlichen Übereinstimmungsmetrik Bestimmen der durchschnittlichen Übereinstimmungsmetrik basierend auf Übereinstimmungsmetriken für jedes Paar einer vordefinierten Anzahl der simulierten EKG-Signale (262-266) umfasst, welche die beste Übereinstimmung in Bezug auf das gemessene EKG-Signal (256) aufweisen.

13. Verfahren nach einem der Ansprüche 1 oder 10 bis 12, wobei der Schritt des Variierens (216) der Position (258) der EKG-Elektroden mindestens eines von Folgenden umfasst:
- Variieren der Position mindestens einer der EKG-Elektroden,
- Entfernen einer der EKG-Elektroden und
- Einbeziehen einer zusätzlichen EKG-Elektrode an einer zusätzlichen Position.

14. System zum Bestimmen eines Herzaktivierungsbereichs eines Herzens (154) eines Patienten (150) basierend auf Elektrokardiographie, EKG, wobei das System (100) Folgendes umfasst:
- mindestens eine Schnittstelle (102, 104), die konfiguriert ist, um
Bilddaten (252) des Herzens (154) des Patienten (150) zu erhalten (202),
Bilddaten (254) eines Oberkörpers (152) des Patienten (150) zu erhalten (254),
Positionen (258) von EKG-Elektroden (142) an dem Oberkörper (152) zu erhalten (208) und
ein gemessenes EKG-Signal (256), das an dem Patienten (150) unter Verwendung der EKG-Elektroden (142) gemessen wird, zu erhalten (206), und
- eine Steuerschaltung (106), die konfiguriert ist, um
ein Modell (260) zum Schätzen elektrischer Potentiale auf der Haut des Oberkörpers (152) des Patienten (150) in Abhängigkeit von der Herzaktivität zu parametrisieren (210), wobei das Modell (260) basierend auf den Bilddaten (252) des Herzens (154), den Bilddaten (254) des Oberkörpers (152), den Positionen (258) der EKG-Elektroden (142) und dem gemessenen EKG-Signal (256) parametrisiert wird,
mehrere simulierte EKG-Signale (262-266) für mehrere Muster der Herzaktivität durch Abtasten der elektrischen Potentiale an dem Oberkörper an den entsprechenden EKG-Elektrodenpositionen unter Verwendung des Modells (260) zu bestimmen (212),
den Herzaktivierungsbereich des Herzens (154) basierend auf Vergleichen der mehreren simulierten EKG-Signale (262-266) mit dem gemessenen EKG-Signal (256) zu bestimmen (218),
**dadurch gekennzeichnet, dass** die Steuerschaltung ferner konfiguriert ist, um eine durchschnittliche Übereinstimmungsmetrik basierend auf den simulierten EKG-Signalen (262-266) und/oder dem gemessenen EKG-Signal (256) zu bestimmen (214), und
Variieren (216) einer Position (258) der EKG-Elektroden in dem Modell und Simulieren (212) von weiteren mehreren simulierten EKG-Signalen (262-266) für die verschiedenen Muster der Herzaktivität unter Verwendung des Modells (260) in Abhängigkeit von der durchschnittlichen Übereinstimmungsmetrik.

15. System nach Anspruch 14, wobei die Steuerschaltung (106) konfiguriert ist, um das Verfahren (200) nach einem der Ansprüche 1 bis 14 durchzuführen.

16. Computerprogrammprodukt, umfassend ein Programm, das direkt in einen Speicher einer programmierbaren Steuerschaltung (106) geladen werden kann, umfassend Programmmittel zum Ausführen aller Schritte des Verfahrens (200) nach einem der Ansprüche 1 bis 13, wenn das Programm in der Steuerschaltung (106) ausgeführt wird.

17. Datenträger, umfassend darauf gespeicherte, elektronisch lesbare Steuerinformationen, der konfiguriert ist, um das Verfahren (200) nach einem der Ansprüche 1 bis 13 durchzuführen, wenn der Datenträger (108) in einer Steuerschaltung (106) verwendet wird.

## Revendications

1. Procédé de détermination d'une zone d'activation cardiaque d'un cœur (154) d'un patient (150) sur la base de l'électrocardiographie, ECG, le procédé (200) comprenant :
- l'obtention (202) de données d'image (252) du cœur (154) du patient (150),
- l'obtention (204) de données d'image (254) d'un torse (152) du patient (150),
- l'obtention (208) de positions (258) d'électrodes ECG (142) au niveau du torse (152),
- l'obtention (206) d'un signal ECG mesuré (256) mesuré chez le patient (150) à l'aide des électrodes ECG (142),
- le paramétrage (210) d'un modèle (260) pour estimer des potentiels électriques sur la peau du torse du patient (150) en fonction de l'activité cardiaque, dans lequel le modèle est paramétré sur la base des données d'image (252) du cœur (154), des données d'image (254) du torse (152), des positions (258) des électrodes ECG (142) et du signal ECG mesuré (256),
- la détermination (212) de multiples signaux ECG simulés (262 à 266) pour de multiples schémas d'activité cardiaque en échantillonnant les potentiels électriques sur le torse au niveau des positions d'électrodes ECG correspondantes à l'aide du modèle (260),
- la détermination (218) de la zone d'activation cardiaque du cœur (154) sur la base de la comparaison des multiples signaux ECG simulés (262 à 266) avec le signal ECG mesuré (256),
**caractérisé en ce que** le procédé comprend également :
- la détermination (214) d'une métrique de correspondance moyenne sur la base des signaux ECG simulés (262 à 266) et/ou du signal ECG mesuré (256),
- en fonction de la métrique de correspondance moyenne, la variation (216) d'une position (258) des électrodes ECG dans le modèle et la simulation (212) de multiples signaux ECG simulés supplémentaires (262 à 266) pour les différents schémas d'activité cardiaque à l'aide du modèle (260).

2. Procédé selon la revendication 1, dans lequel ledit paramétrage (210) du modèle (260) est basé sur un modèle générique configuré pour fournir des signaux ECG sur la base au moins des éléments suivants
- une taille du cœur (154),
- une géométrie du cœur (154),
- une taille du torse (152),
- une géométrie du torse (152),
- une disposition du cœur (154) par rapport au torse (152), et
- une disposition des positions (258) des électrodes (142).

3. Procédé selon la revendication 2, dans lequel le modèle générique est configuré pour estimer des potentiels électriques sur le torse (152) sur la base d'un modèle informatique d'électrophysiologie.

4. Procédé selon la revendication 2 ou la revendication 3, dans lequel le modèle générique comprend une représentation maillée du cœur (154) et/ou du torse (152), dans lequel chaque sommet de maillage étant associé à au moins un des éléments suivants
- un potentiel électrique estimé correspondant,
- une vitesse de conduction du tissu correspondant, et
- une diffusivité électrique du tissu correspondant.

5. Procédé selon l'une quelconque des revendications 2 à 4, dans lequel le paramétrage (210) du modèle (260) sur la base du modèle générique comprend au moins un des éléments suivants :
- la variation d'une vitesse de conduction et/ou d'une diffusivité électrique du tissu cardiaque jusqu'à ce qu'un complexe QRS des signaux ECG simulés (262 à 266) corresponde à un complexe QRS du signal ECG mesuré (256), et
- la variation de la conductivité électrique des tissus du torse jusqu'à ce que des amplitudes de signal d'au moins certains des signaux ECG simulés (262 à 266) correspondent à des amplitudes de signal du signal ECG mesuré correspondant (256).

6. Procédé selon l'une quelconque des revendications 2 à 5, dans lequel le paramétrage (210) du modèle (260) sur la base du modèle générique comprend au moins un des éléments suivants :
- la variation d'une orientation du cœur (154) par rapport au torse (152) afin d'optimiser une correspondance entre au moins certains des signaux ECG simulés (262 à 266) et le signal ECG mesuré correspondant (256), et
- la variation d'un placement du cœur (154) par rapport au torse (152) afin d'optimiser une correspondance entre au moins certains des signaux ECG simulés (262 à 266) et le signal ECG mesuré correspondant (256).

7. Procédé selon l'une quelconque des revendications 2 à 6, dans lequel le modèle générique est configuré pour estimer des potentiels électriques sur le torse (152) sur la base d'un modèle basé sur les données.

8. Procédé selon l'une quelconque des revendications précédentes, dans lequel les différents schémas d'activité cardiaque comprennent au moins un des éléments suivants :
- une activation normale du myocarde débutant dans l'endocarde septal,
- l'activation précoce dans un ensemble prédéfini de points endocardiques septaux,
- l'activation précoce dans un ensemble prédéfini de points endocardiques non septaux, et
- l'activation précoce dans un ensemble prédéfini de points non endocardiques.

9. Procédé selon l'une quelconque des revendications précédentes, dans lequel le signal ECG (256, 262 à 266) comprend un ECG à 12 dérivations.

10. Procédé selon la revendication 1, comprenant également :
- la répétition des étapes de détermination (214) de la métrique de correspondance moyenne, de variation (216) de la position des électrodes ECG et de simulation (212) de multiples signaux ECG supplémentaires (262 à 266) si (220) la métrique de correspondance moyenne dépasse un seuil prédéfini.

11. Procédé selon la revendication 1 ou la revendication 10, dans lequel la détermination (214) de la métrique de correspondance moyenne comprend la détermination de la métrique de correspondance moyenne sur la base de métriques de correspondance pour chaque paire des signaux ECG simulés (262 à 266)

12. Procédé selon l'une quelconque des revendications 1, 10 ou 11, dans lequel la détermination (214) de la métrique de correspondance moyenne comprend la détermination de la métrique de correspondance moyenne sur la base de métriques de correspondance pour chaque paire d'un nombre prédéfini des signaux ECG simulés (262 à 266) ayant la meilleure correspondance par rapport au signal ECG mesuré (256).

13. Procédé selon l'une quelconque des revendications 1 ou 10 à 12, dans lequel l'étape de variation (216) de la position (258) des électrodes ECG comprend au moins une des étapes suivantes :
- la variation de la position d'au moins une des électrodes l'ECG,
- le retrait d'une des électrodes de l'ECG, et
- l'inclusion d'une électrode ECG supplémentaire au niveau d'une position supplémentaire.

14. Système de détermination d'une zone d'activation cardiaque d'un cœur (154) d'un patient (150) sur la base de l'électrocardiographie, ECG, le système (100) comprenant :
- au moins une interface (102, 104) configurée pour obtenir (202) des données d'image (252) du cœur (154) du patient (150),
obtenir (254) des données d'image (254) d'un torse (152) du patient (150),
obtenir (208) des positions (258) d'électrodes ECG (142) au niveau du torse (152), et
obtenir (206) un signal ECG mesuré (256) mesuré chez le patient (150) à l'aide des électrodes ECG (142), et
- un circuit de commande (106) configuré pour
paramétrer (210) un modèle (260) pour estimer des potentiels électriques sur la peau du torse (152) du patient (150) en fonction de l'activité cardiaque, dans lequel le modèle (260) est paramétré sur la base des données d'image (252) du cœur (154), des données d'image (254) du torse (152), des positions (258) des électrodes ECG (142) et du signal ECG mesuré (256),
déterminer (212) de multiples signaux ECG simulés (262 à 266) pour de multiples schémas d'activité cardiaque en échantillonnant les potentiels électriques sur le torse au niveau des positions d'électrodes ECG correspondantes à l'aide du modèle (260),
déterminer (218) la zone d'activation cardiaque du cœur (154) sur la base de la comparaison des multiples signaux ECG simulés (262 à 266) avec le signal ECG mesuré (256),
**caractérisé en ce que** le circuit de commande est également configuré pour déterminer (214) une métrique de correspondance moyenne sur la base des signaux ECG simulés (262 à 266) et/ou du signal ECG mesuré (256), et
en fonction de la métrique de correspondance moyenne, la variation (216) d'une position (258) des électrodes ECG dans le modèle et la simulation (212) de multiples signaux ECG simulés supplémentaires (262 à 266) pour les différents schémas d'activité cardiaque à l'aide du modèle (260).

15. Système selon la revendication 14, dans lequel le circuit de commande (106) est configuré pour réaliser le procédé (200) selon l'une quelconque des revendications 1 à 14.

16. Produit de programme informatique comprenant un programme qui peut être chargé directement dans une mémoire d'un circuit de commande programmable (106), comprenant des moyens de programme pour exécuter toutes les étapes du procédé (200) selon l'une quelconque des revendications 1 à 13 lorsque le programme est exécuté dans le circuit de commande (106).

17. Support de données comprenant des informations de commande lisibles électroniquement stockées sur celui-ci, qui est configuré pour réaliser le procédé (200) selon l'une quelconque des revendications 1 à 13 lorsque le support de données (108) est utilisé dans un circuit de commande (106).
